(19) Europäisches Patentamt
European Patent Office
Office européen des brevets

(11) Publication number: **0 461 155 B1**

(12) # EUROPEAN PATENT SPECIFICATION

(45) Date of publication of patent specification: **27.07.94**

(51) Int. Cl.[5]: **C12N 15/10**, C12Q 1/68

(21) Application number: **90904029.7**

(22) Date of filing: **15.02.90**

(86) International application number:
**PCT/US90/00942**

(87) International publication number:
**WO 90/10064 (07.09.90 90/21)**

(54) **IMPROVED METHODS FOR IN VITRO DNA AMPLIFICATION AND GENOMIC CLONING AND MAPPING.**

(30) Priority: **03.03.89 US 318385**

(43) Date of publication of application:
**18.12.91 Bulletin 91/51**

(45) Publication of the grant of the patent:
**27.07.94 Bulletin 94/30**

(84) Designated Contracting States:
**AT BE CH DE DK ES FR GB IT LI LU NL SE**

(56) References cited:
**EP-A- 0 200 362**

**Gene, vol. 34, no. 2/3, 1985, Elsevier Science Publishers, (Amsterdam, NL), C. Coleclough et al., pp. 305-314**

(73) Proprietor: **GENENTECH, INC.**
**460 Point San Bruno Boulevard**
**South San Francisco California 94080(US)**

(72) Inventor: **MILLER, Harvey, I.**
**225 Linda Lane**
**Pleasant Hill, CA 94523(US)**

(74) Representative: **Nicholls, Kathryn Margaret et al**
**MEWBURN ELLIS**
**York House**
**23 Kingsway**
**London WC2B 6HP (GB)**

**EP 0 461 155 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Background of the Invention

The field of this invention is the in vitro amplification of nucleic acids. In particular, this invention relates to novel methods for cloning and replicating nucleic acids in the course of sequencing and physical mapping. It also relates to efficient methods for physical mapping of highly complex and lengthy genomes.

The ability to map and sequence genomic DNA has important applications in the biological and medical sciences. The localization of human genes responsible for rare diseases is an important starting point for gene isolation and cloning, and the identification of relevant gene products and mutations, leading ultimately to improved understanding of the molecular basis of disease. By identifying genes or regions of human chromosomal DNA involved in hereditary forms of cancer, Alzheimer's disease, and other diseases, new methods of diagnosis and treatment may be developed.

Mapping a genome refers to pinpointing the location of genes and other features of interest on particular chromosomes. Sequencing refers to determining the order of nucleotides on the chromosomes. The two main types of genome maps are genetic linkage maps and physical maps. Genetic linkage maps are generally made by studying the frequency with which two different traits are inherited together, or linked. Physical maps are derived mainly from chemical measurements made on the DNA molecules that comprise the genome. Accordingly, physical maps can be of several different types and include restriction maps as well as lower resolution maps obtained by in situ hybridization. All these maps share the common goal of placing information about genes in a systematic linear order according to their relative positions along a chromosome.

Restriction maps are based on sites in DNA that are cut by special proteins called restriction enzymes. Each enzyme recognizes a specific short sequence of nucleotides termed a "recognition sequence" and cuts each strand of the DNA at some point, termed a "cleavage site" or "restriction site," that is within the recognition sequence or some distance away from it. Since many different nucleotide sequences are recognized by one or another restriction enzyme, and those sequences are generally dispersed randomly throughout a genome, a physical map may be constructed by determining the relative locations of different restriction sites precisely.

The genome of the bacterium E. coli comprises about 4.7 million base pairs. The smallest human chromosome is ten times that size, with the complete (haploid) human genome comprising about 3 billion base pairs. Because of the large size of most genomes, the restriction enzymes that are of particular value in restriction mapping are those having recognition sites that occur relatively infrequently, or are otherwise distantly spaced throughout the genome, such that the genome may be cut into relative large DNA fragments, preferably 100,000 to 2 million or more base pairs long. The fragments of DNA that are produced upon digestion of a DNA substrate with a restriction enzyme are termed "restriction fragments." In general, the fewer the number of genomic restriction fragments generated, the easier is the task of correctly ordering them in a physical map.

The largest genome that has been mapped with restriction enzymes that cleave DNA infrequently is the single chromosome of E. coli. Smith, et al., Science 236:1448-1453 (1987). The approach taken in that instance was to digest intact E. coli chromosomal DNA with the restriction enzyme *Not*I, which cuts within an eight nucleotide recognition sequence, thereby producing a range of DNA fragments from 15,000 base pairs to 1 million base pairs. Most of the information needed to order the DNA restriction fragments came from hybridization studies using labelled probes corresponding to E. coli genes that had previously been cloned and characterized.

Thus, the first step toward constructing a physical map of a genome generally involves the isolation and cloning of discrete genomic DNA fragments. In theory, sensitive DNA-probe technologies make it possible to construct physical maps while cloning only a small fraction of the genome that is being mapped. In practice, however, such an approach is suitable only for the coarsest level of physical mapping. At higher resolutions, most physical mapping is likely to be carried out by analyzing a collection of overlapping DNA clones that cover the entire genome. Physical mapping would entail the ordering of the individual DNA clones according to their positions in the original genome. The individual clones are especially useful because they provide an inexhaustible source of the DNA from each genomic region. Having available a collection of genomic DNA clones is also a prerequisite to determining the nucleotide sequence of the genome, since the clones would provide the actual DNA fragments that would be purified and prepared for sequencing.

The principal limitation on the construction of a high-resolution physical map of a genome from an ordered collection of DNA clones is the failure of present methods to provide the means for cloning very

large DNA fragments. Using standard recombinant DNA techniques, for example, the size of the largest DNA fragment that may be clonally propagated in a bacterial host cell is about 50,000 base pairs or less. That result is typically achieved by splicing the DNA fragment into a cosmid -- a modified bacteriophage lambda vector specifically designed to accommodate DNA inserts on the order of 40,000 to 50,000 base pairs in length.

The shortcomings of this cosmid cloning system in constructing a physical map of a genome are two-fold. First, very large numbers of cosmid clones must be constructed to generate a complete set of mutually overlapping DNA fragments for the entire genome. In the case of the haploid human genome, for example, it has been estimated that anywhere from 75,000 to 375,000 or more different cosmid clones would be required for a high-resolution physical map. Pines, 1987, "Mapping the Human (Howard Hughes Medical Institute, Bethesda, MD). Second, cosmid clones are known to frequently accumulate deletions which may result from selection for a shorter size for faster replication and/or metabolic imbalances caused by the increased dosage of a particular gene on the cosmid that affects the growth of the bacterial host cells. Therefore, cosmid clones, especially those containing essential genes, are usually difficult to maintain.

Recently, a method has been described for cloning large fragments of exogenous DNA into yeast by means of artificial chromosome vectors. Burke, et al., 1987, Science 236:806-812. The use of this "yeast artificial chromosome" (YAC) cloning system offers a significant advantage over the cosmid cloning system in that genomic DNA fragments that range in size up to several hundred thousand base pairs may be successfully propagated. There are, however, several limitations on the use of the YAC cloning system, particularly as it may be applied to genome mapping. For example, both the number of cloned molecules per yeast cell and the number of yeast cells per colony are much lower in YAC cloning than in cosmid cloning in bacteria, thus limiting the quantities of cloned DNA available for analysis. Furthermore, as with any in vivo cloning method, the DNA fragments cloned in YAC vectors can undergo rearrangements or deletions in the host cell that may make it difficult to obtain a set of clones representing the entire genome.

It is one object of the present invention, therefore, to provide a method for efficiently cloning large fragments of genomic DNA that avoids the problems inherent in the existing in vivo cloning technology. It is a further object of the present invention to provide a method for amplifying a desired nucleic acid sequence, such as that represented in a cloned genomic DNA fragment, thereby producing sufficient quantities of the sequence for physical or chemical analysis. The ability to carry out this method in vitro makes it generally useful for amplifying nucleic acids from any source, without the problems of selectivity, rearrangements, and deletions that may result from propagating an exogenous nucleic acid sequence in a host cell. At the same time, the ability to efficiently amplify nucleic acid sequences ranging in size up to several hundred thousand base pairs or more makes the method ideally suited for such applications as constructing a high-resolution physical map of a complex genome or isolating intact medically or biologically important genes or gene clusters.

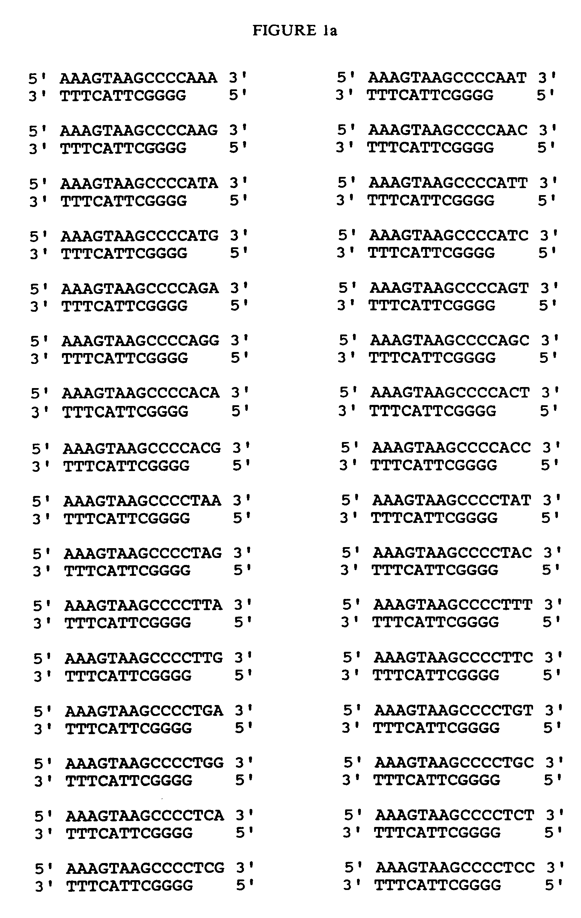

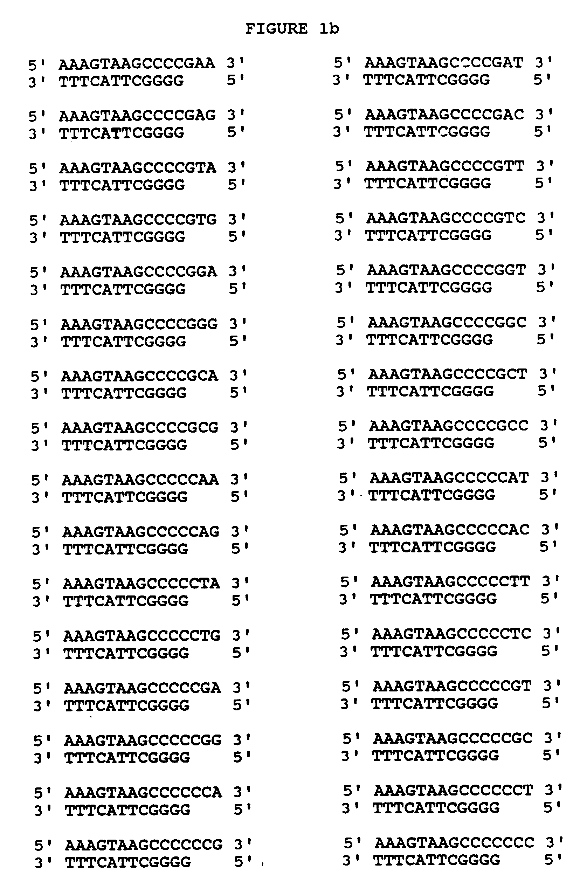

Figures 1a-1b (referred to hereafter as Figure 1) show 64 oligonucleotide linkers having cohesive end sequences complementary to each of the possible restriction fragment cohesive ends resulting from digestion of a genomic DNA substrate with the restriction endonuclease *Sfi* I.

The present invention is directed to an improved method for amplification of DNA that entails the synthesis of novel replicable DNAs comprising a bacteriophage phi29 replication origin and genomic DNA heterologous to phi29, and its use in conjunction with improved methods for cloning genomic DNA and for mapping genomic DNA.

The method for amplification of a nucleic acid sequence comprises the steps of:

(a) synthesizing a double-stranded nucleic acid, comprising a bacteriophage phi29 replication origin and a nucleotide sequence which is heterologous to phi29; and

(b) synthesizing DNA from said double-stranded nucleic acid under the control of said replication origin.

The method for cloning genomic DNA comprises the steps of:

(a) digesting genomic DNA with a restriction enzyme that generates multiple different cohesive-end sequences;

(b) contacting the digested genomic DNA with a linker, said linker comprising a reporter nucleotide sequence comprising a bacteriophage phi29 replication origin and a cohesive-end sequence complementary to a cohesive-end sequence generated by the restriction enzyme of step (a), under conditions such that a linker is joined to a restriction fragment of genomic DNA; and

(c) amplifying the product of step (b) using bacteriophage phi29 DNA polymerase.

The method for mapping genomic DNA restriction fragments comprises the steps of:

(a) digesting genomic DNA with a restriction enzyme that generates multiple different cohesive-end sequences;

(b) amplifying the restriction fragment products of step (a) by

(1) providing a library of linkers, wherein:

each linker comprises a bacteriophage phi29 replication origin and a cohesive-end sequence, and

the library comprises linkers having cohesive-end sequences complementary to substantially every cohesive-end sequence generated by the restriction enzyme of step (a);

(2) contacting the restriction fragments of step (a) with linkers from said library under conditions such that each restriction fragment cohesive-end is joined with a linker; and

(3) contacting the products of step (2) with bateriophage phi29 DNA polymerase, bacteriophage phi29 DNA synthesis priming protein, and four different nucleoside triphosphates under conditions such that multiple copies of each restriction fragment are synthesized under the control of said bacteriophage phi29 replication origin;

(c) determining the cohesive-end sequence of each of said restriction fragments; and in accordance therewith,

(d) aligning the restriction fragments in a physical map.

The advantages obtained by utilizing the methods of the present invention for cloning genomic DNA and mapping genomic DNA restriction fragments as compared to methods already known in the art is that the present invention allows the cloning and mapping of genomic DNA of virtually any length, without resort to the burdensome and error-prone steps of inserting the genomic DNA into specialized vectors and replicating it in vivo, and without the need for prior knowledge of the nucleotide sequence or genetic organization of any part of the genomic DNA.

The advantage obtained by the method for amplification of a heterologous DNA under the control of a bacteriophage phi29 replication origin is that the in vitro synthesis of DNA products, in the presence of phi29 DNA polymerase and deoxyribonucleoside triphosphates, occurs continuously, using as a template DNA up to 100,000 base pairs or more in length. The method of the present invention therefore avoids the multiple exacting primer hybridization reactions required by the polymerase chain reaction (PCR) method of Mullis, et al., U.S. Pat. No. 4,683,195, that is commonly used in the art, as well as the inherent limitations of the PCR method as applied to the amplification of lengthy DNA substrates.

The term "genomic DNA" as used herein refers to any DNA comprising a sequence that is normally present in the genome of a prokaryotic or eukaryotic cell or a virus. The genomic DNA of a eukaryotic cell includes, for example, nuclear and extranuclear chromosomal DNA, such as that present in mitochondria and chloroplasts. Also included within the scope of the term "genomic DNA" is any cDNA prepared from a messenger RNA or from the RNA genome of a virus. Methods for the extraction and/or purification of genomic DNA have been described, for example, by Gross-Bellard, et al., 1978, Eur. J. Biochem. 36:32-38, Smith, et al., 1987, Meth. Enzymol. 151:461-489, and Moon, et al., 1987, Nuc. Acids Res. 15:611-630. Methods for the preparation of cDNA are well known in the art. See, for example, Maniatis, et al., Molecular Cloning: A Laboratory Manual (New York, Cold Spring Harbor Laboratory, 1982).

The term "heterologous" as used herein in reference to a nucleic acid sequence refers to a nucleic acid sequence not ordinarily replicated under the control of a bacteriophage phi29 replication origin. Typically the heterologous nucleic acid sequence will comprise a DNA sequence present in the nuclear or extranuclear genome of a prokaryotic or eukaryotic organism, or the genome of a virus other than bacteriophage phi29. The heterologous nucleic acid sequence may be prepared by any suitable method, such as recovery from a naturally occurring nucleic acid by use of a restriction enzyme, or in vitro synthesis, including, for example, the synthesis of a complementary DNA (cDNA) from an RNA.

The term "reporter sequence" as used herein refers to any nucleic acid sequence by which it is possible to identify, either directly or indirectly, another nucleic acid molecule with which the reporter sequence is associated. The reporter sequence may be of defined sequence or defined function or both. For example, if the reporter sequence comprises a defined nucleotide sequence, the nucleic acid molecule with which it is associated may be identified by a hybridization method, wherein the reporter sequence hybridizes with a complementary nucleic acid that is immobilized on a solid support. The hybridization method may be carried out by any suitable method, including those described by Choutelle, et al., 1978, Gene 3:113-122, and European Pat. App. 0 221 308 (Carrico). Alternatively, the reporter sequence may be detected by its function, with or without knowledge of its sequence. Examples include the gene for a detectable phenotype, or preferably a replication origin or a promoter. If the reporter sequence comprises a replication origin or a promoter, for example, the nucleic acid molecule with which the reporter sequence is associated is readily identified on the basis of its replication or transcription under the control of the reporter sequence.

The term "cohesive-end" as used herein in reference to linkers and restriction fragments refers to a single-strand extension at the end of a double-stranded nucleic acid molecule that extends beyond the region of base-pairing between the complementary strands. Such single-strand extensions are termed

cohesive ends because they are capable of hybridizing with one another sequence through base-pairing of complementary nucleotides, thereby facilitating the intramolecular or intermolecular joining of nucleic acids. The nucleotide sequence of the single-strand extension is termed the "cohesive-end sequence." Restriction fragments having cohesive ends are preferably obtained by digestion of double-stranded DNA with a suitable restriction enzyme, such as any of those hereinafter disclosed. Linkers having cohesive ends may be obtained by the same method, or may be produced synthetically, such as by the annealing of single-stranded oligonucleotides.

The term "oligonucleotide" as used herein in reference to linkers and primers is defined as a nucleic acid molecule comprised of two or more deoxyribonucleotides or ribonucleotides. A desired oligonucleotide may be prepared by any suitable method, such as purification from a naturally occurring nucleic acid or de novo synthesis. Several methods have been described in the literature, for example, for the synthesis of oligonucleotides of defined sequence using various techniques in organic chemistry. Narang, et al., 1979, Meth. Enzymol. 68:90-109; Caruthers, et al., 1985, Meth. Enzymol. 154:287; Froehler, et al., 1986, Nuc. Acids Res. 14:5399-5407. Oligonucleotides prepared by any method may subsequently be joined together by ligation or otherwise to form a single oligonucleotide of any required length and sequence.

The term "linker" as used herein refers to an oligonucleotide, whether occurring naturally or produced synthetically, which comprises double-stranded DNA.

The term "primer" as used herein refers to an oligonucleotide, whether occurring naturally or produced synthetically, which is substantially complementary or homologous to all or part of a nucleic acid sequence to be amplified. The primer must be sufficiently long to hybridize with a template nucleic acid comprising the sequence to be amplified, and to prime the synthesis of an extension product in the presence of an agent for polymerization. Typically the primer will contain 15-30 or more nucleotides, although it may contain fewer nucleotides. It is not necessary, however, that the primer reflect the exact sequence of the nucleic acid sequence to be amplified or its complement. For example, non-complementary bases can be interspersed into the primer, or complementary bases deleted from the primer provided that the primer is capable of hybridizing with the nucleic acid sequence to be amplified or its complement, under the conditions chosen.

The term "origin-primer" as used herein refers to an oligonucleotide whether occurring naturally or produced synthetically which comprises a replication origin, as defined further herein, joined to the 5' end of a primer. Under suitable conditions and in the presence of an agent for polymerization, the origin primer is capable of acting as a point of initiation of an origin-primer extension product that includes the nucleic acid sequence to be amplified or its complementary sequence, and the replication origin of the origin-primer.

The term "secondary primer" as used herein refers to an oligonucleotide whether occurring naturally or produced synthetically, which under suitable conditions and in the presence of an agent for polymerization, is capable of acting as a point of initiation for a secondary primer extension product that includes the nucleic acid sequence to be amplified or its complementary sequence.

The term "extension product" as used herein refers to a nucleic acid molecule, the synthesis of which is initiated at the 3'-OH terminus of a primer, using as a template for synthesis the nucleic acid molecule to which the primer is hybridized.

The term "agent for polymerization" as used herein is generally understood to refer to any enzyme that catalyzes the synthesis of a nucleic acid molecule from deoxyribonucleotides or ribonucleotides, using an existing nucleic acid as a template. Examples of such enzymes include DNA polymerase, RNA polymerase, and reverse transcriptase.

In one of its aspects, the present invention is a method for cloning genomic DNA which has as its initial step digesting genomic DNA with a restriction enzyme that generates multiple different cohesive-end sequences. Such enzymes are well known in the art and include, for example, the restriction enzymes *Bgl* I, *Bst*X I, *Hga* I, and *Sfi* I.

The predictable cleavage of double-stranded DNA by a restriction enzyme results from the enzyme's recognition of a certain sequence of base pairs (recognition sequence) in the DNA substrate. For any individual restriction enzyme, this specificity is characteristic, and essentially invariant among DNAs. The recognition sequences for most restriction enzymes consist of a specific sequence of four to eight base pairs or more, and may additionally include one or more random base pairs.

In addition to the recognition sequence, a restriction enzyme is also characterized by the sites at which it introduces breaks in the phosphodiester bonds of each strand of the DNA substrate (cleavage sites) to generate discrete restriction fragments. The cleavage site for a particular restriction enzyme may occur within the enzyme's recognition sequence or some distance away from it. In the case of a restriction enzyme that is capable of generating multiple different cohesive-end sequences, the sequence immediately surrounding the cleavage sites necessarily includes one or more random base pairs.

By way of example, the recognition sequence for the restriction enzyme *Sfi* I comprises a specific sequence of four base pairs separated by five random base pairs from a specific sequence of four base pairs as shown:

```
                 ↓
5' . . . GGCCNNNNNGGCC . . . 3'
3' . . . CCGGNNNNNCCGG . . . 5'
             ↑
```

wherein N may be any one of the four nucleotides A (adenine), T (thymine), G (guanine), or C (cytosine). *Sfi*I introduces staggered cleavages within the recognition sequence at the sites indicated by the vertical arrows. Accordingly, each resulting *Sfi*I restriction fragment terminates at one or both ends with a cohesive end having the structure

```
5' . . . GGCCNNNN 3'
3' . . . CCGGN    5'
```

wherein the cohesive-end sequence, 5'-NNN-3', comprises any one of 64 possible trinucleotide sequences.

In general, if the substrate DNA is a linear molecule, the restriction fragments resulting from digestion of the substrate DNA with a restriction enzyme that generates multiple different cohesive-end sequences will be of two types: restriction fragments that comprise one of the original ends of the linear substrate DNA and one restriction enzyme-generated cohesive end, and so-called "internal" restriction fragments that comprise two restriction enzyme-generated cohesive ends. If the substrate DNA is circular, all the restriction fragments will comprise two restriction enzyme-generated cohesive ends.

As will be apparent from this description, both the number of different restriction fragments that are produced upon complete digestion of the substrate DNA with a restriction enzyme and the number of different cohesive-end sequences that are possible for those restriction fragments will depend upon the restriction enzyme that is used.

The number of different restriction fragments that are produced upon complete digestion of the substrate DNA with a restriction enzyme in turn depends upon the number of occurrences of the restriction enzyme recognition sequence in the substrate DNA. The number of occurrences of a specific recognition sequence may be estimated from the length of the restriction enzyme recognition sequence and the size of the substrate DNA. Given that DNA is comprised of four different nucleotides, the statistical frequency of a specific recognition sequence of length n base pairs is given by the formula:

$$\text{frequency} = \frac{1}{4^n}$$

For example, a recognition sequence of four base pairs is expected to occur with a frequency of 1/256, a recognition sequence of five base pairs is expected to occur with a frequency of 1/1024, a recognition sequence of six base pairs is expected to occur with a frequency of 1/4096, and so on. The expected number of occurrences of a specific recognition sequence in the substrate DNA is then obtained by multiplying the calculated recognition sequence frequency by the size of the substrate DNA in base pairs.

The number of different cohesive-end sequences that are possible among the restriction fragments produced by a restriction enzyme capable of generating multiple different cohesive-end sequences is a function of the number of allowed nucleotide variations within the cohesive-end sequence. Most commonly, each of the random nucleotides within the cohesive-end sequence may be any one of the four nucleotides adenine, thymine, guanine, or cytosine, in which case the expected number of different cohesive-end sequences is equal to $4^n$, wherein n is the number of random nucleotides within the cohesive-end sequence.

Any restriction enzyme capable of generating multiple different cohesive-end sequences may be used in the present invention, although the choice of a restriction enzyme in a particular instance will preferably be made on the basis of the size and complexity of the substrate DNA.

In general, it is desirable that the recognition sequence of the restriction enzyme chosen occur relatively infrequently in the substrate DNA, in order to reduce the number of DNA restriction fragments that

are produced and that are subject to subsequent manipulation and/or analysis. In mapping studies of prokaryotic or eukaryotic chromosomal DNA, for example, a restriction enzyme having a recognition sequence of at least six base pairs will usually be required, preferably at least eight base pairs, in order that the resulting restriction fragments have an average size within the range of about 10,000 base pairs to 100,000 base pairs or more. Optionally, the number of restriction fragment products may be reduced (and their average size correspondingly increased) by adjusting the conditions under which the substrate DNA is contacted with the restriction enzyme (e.g., reaction time or temperature), such that the DNA substrate is only partially digested, with one or more of the restriction enzyme cleavage sites remaining uncleaved.

At the same time, it is desirable that the restriction enzyme be capable of generating restriction fragments of the substrate DNA having a sufficient number of different cohesive-end sequences. Generally, the greater the number of cohesive-end sequences generated by the restriction enzyme, the greater the likelihood of distinguishing the resulting restriction fragments from one another on the basis of their cohesive-end sequences, and in turn, the easier it will be to clone the individual DNA restriction fragments and/or to order them in a physical map. For the digestion of prokaryotic or eukaryotic chromosomal DNA, for example, the restriction enzyme chosen will usually be one capable of generating at least 64 different cohesive-end sequences, which corresponds to a cohesive-end sequence comprising a minimum of three random nucleotides.

After digesting genomic DNA with a suitable restriction enzyme, the method for cloning genomic DNA has as its next step contacting the resulting DNA restriction fragment(s) with an oligonucleotide linker comprising a reporter sequence and a cohesive-end sequence complementary to one of the multiple different cohesive-end sequences generated by the restriction enzyme.

The oligonucleotide linker and the DNA restriction fragment(s) are desirably incubated together under conditions such that the cohesive-end of the oligonucleotide linker hybridizes specifically with the complementary cohesive-end of a restriction fragment. The linker and a DNA restriction fragment with which it has hybridized are then covalently joined using any suitable method, preferably by ligation with DNA ligase. The presence of the reporter sequence provides the means for identifying the resulting product from amongst a mixture of DNA restriction fragments, and preferably provides the means for isolating and/or selectively amplifying the resulting product.

If digestion of the genomic DNA produces a mixture of different restriction fragments comprising different cohesive-end sequences, each different restriction fragment of the mixture conveniently may be cloned according to the present invention by contacting the mixture with a combination of two or more linkers having cohesive-end sequences complementary to those of the different restriction fragments.

Because the cohesive-end sequences of the different restriction fragments of the mixture usually will not be known with certainty, it is desirable under such circumstances that the combination of linkers comprise a library of linkers having cohesive-end sequences complementary to substantially every possible cohesive-end sequence generated by the particular restriction enzyme used. In this manner, it may be assured that substantially every restriction fragment cohesive-end is joined with a linker. Depending on the number of different restriction fragments resulting from digestion of the genomic DNA, it may further be desirable that individual aliquots of the mixture be separately contacted with each individual linker from a library of linkers, preferably two or more linkers comprising different cohesive-end sequences, in separate reaction vessels, as for example, the separate wells of a microtiter plate. In this manner, the number of different restriction fragments which become joined with linkers in any one reaction may be reduced, in turn reducing or eliminating the need in a subsequent step to separate each different restriction fragment, one from another, in order to achieve the cloning of each individual genomic DNA restriction fragment.

The final step of the method for cloning genomic DNA is the amplification of a genomic DNA restriction fragment to which a linker and reporter sequence has been joined either in vivo, such as by replication in a host cell, or in vitro. Known methods of in vitro amplification are described in, for example, Mullis, et al., supra, or Miller, et al., PCT Publ. No. WO89/06700, published 27 July 1989, using a primer comprising a sequence complementary to that of the reporter sequence. However, neither of these methods is particularly well-suited for the amplification of lengthy DNA fragments. For example, the method of Mullis, et al. is generally limited to the amplification of DNA fragments no larger than several thousand base pairs due to the low processivity and relatively high error rate of the DNA polymerase used in the amplification reaction. Higuchi, et al., 1988, Nuc. Acids Res. 16:7351-7367; Saiki, et al., 1988, Science 239:487-491. Furthermore, the method of Mullis, et al. requires multiple cycles of nucleic acid denaturation and primer hybridization, resulting in burdensome labor or automation costs.

Accordingly in preferred embodiments of the present invention, the reporter sequence used in the cloning of genomic DNA comprises a bacteriophage phi29 replication origin and amplification of the restriction fragment to which it is joined is accomplished in vitro using bacteriophage phi29 DNA

polymerase.

The term "replication origin" generally refers to a sequence in DNA at which DNA synthesis is initiated by an agent for polymerization. Gutierrez, et al., 1988, Nuc. Acids Res., 16:5895-5914, disclose that the minimal phi29 replication origins utilized by phi29 DNA polymerase are located within the terminal 12 base pairs at each end of phi29 DNA. The so-called "left origin of replication" comprises the sequence 5'-AAAGTAAGCCCC-3' and the "right origin of replication" comprises the sequence 5'-AAAGTAGGGTAC-3', with replication proceeding in the 5' - 3' direction as shown. However, because nucleotide changes may be made at one or more positions within these sequences without abolishing their function, all such sequence variants are likewise included within the scope of the term "bacteriophage phi29 replication origin" as used herein.

Blanco, et al., 1984, Gene 29:33-40, and Blanco and Salas, 1984, Proc. Nat. Acad. Sci 81:5325-5329, together describe the purification of bacteriophage phi29 DNA polymerase from E. coli cells transformed with a recombinant plasmid containing the phi29 DNA polymerase gene. Garcia, et al., 1983, Gene 21:65-76, and Prieto, et al., 1984, Proc. Nat. Acad. Sci 81:1639-1643, together describe the purification of bacteriophage phi29 terminal protein from E. coli cells transformed with a recombinant plasmid containing the phi29 terminal protein gene.

Blanco and Salas, 1985, Proc. Nat. Acad. Sci. 82:6404-6408, and Blanco, et al., 1988, in "EMBO Workshop - Gene Organization and Expression in Bacteriophages," at p.63, describe the replication of bacteriophage phi29 DNA in the presence of purified phi29 terminal protein and phi29 DNA polymerase. The replication of double-stranded bacteriophage phi29 DNA is initiated at the replication origins present at both ends of the DNA by a protein priming mechanism. In the presence of phi29 terminal protein, phi29 DNA polymerase and the four deoxynucleoside triphosphates, a terminal protein-dAMP initiation complex is formed that can be elongated to full-length phi29 DNA by phi29 DNA polymerase by a mechanism of strand displacement.

In another of its aspects, therefore, the present invention provides a method for amplifying heterologous DNA sequences on the order of 10,000 base pairs to 100,000 base pairs or more in length, as well as heterologous DNA sequences less than 10,000 base pairs in length, which method utilizes a bacteriophage phi29 replication origin and highly processive bacteriophage phi29 DNA polymerase. In accordance with the present invention, it is now feasible to amplify any DNA sequence by incorporating at one or both ends thereof a bacteriophage phi29 replication origin. Thus, the subject invention provides new DNA constructs that do not naturally occur and that can be replicated under the control of a bacteriophage phi29 replication origin.

The bacteriophage phi29 replication origin may be obtained from natural sources or may be produced synthetically, and may be joined to the end of a heterologous DNA fragment in a ligation reaction with DNA ligase or otherwise introduced adjacent to a DNA sequence to be amplified by methods known in the art, such as site directed mutagenesis, or through the use of an origin-primer according to the methods generally described by Mullis, et al., supra.

Amplification of a heterologous DNA sequence under the control of a bacteriophage phi29 replication origin typically will be performed in vitro in a buffered aqueous solution, in the presence of bacteriophage phi29 terminal protein, bacteriophage phi29 DNA polymerase, and the four deoxynucleoside triphosphates, and under suitable conditions of temperature, salts concentration (e.g., $Mg^{2+}$, $NH_4^+$), and pH such that a multiplicity of full length copies of the heterologous DNA are synthesized.

Because replication from a bacteriophage phi29 replication origin proceeds unidirectionally, using as a template one strand of a double-stranded DNA, the amplification of a double-stranded heterologous DNA is accomplished by joining to both DNA ends a linker comprising a bacteriophage phi29 replication origin, such that both strands of the DNA template are replicated. Accordingly, the amplification of a genomic DNA restriction fragment is preferably accomplished by joining to both ends of the restriction fragment linkers comprising a bacteriophage phi29 replication origin. A linker comprising a bacteriophage phi29 replication origin is conveniently referred to as a "phi29 origin linker."

In one embodiment of the invention, the genomic DNA restriction fragment will comprise two restriction enzyme-generated cohesive ends and the phi29 origin linkers preferably will each have a cohesive-end sequence complementary to one of the ends of the genomic DNA restriction fragment. In another embodiment of the invention the genomic DNA restriction fragment will comprise one of the original ends of the genomic DNA substrate and one restriction enzyme-generated cohesive end. Typically the original end of the genomic DNA substrate will be a blunt end, in which case amplification of the genomic DNA restriction fragment preferably will be accomplished by joining to the original end a phi29 origin linker having a blunt end, and joining to the restriction-enzyme generated cohesive end a phi29 origin linker having a complementary cohesive end. The term "blunt end" as used herein refers to an end of a double-stranded nucleic acid that lacks any single-stranded overhang. If the original end of the genomic DNA substrate is not a blunt end, it is preferably converted to a blunt end, for example, by removal of any single-stranded overhang with S1 nuclease, or another suitable single-stranded exonuclease, or by filling in the original ends according to known methods. See, for example, Maniatis, et al., supra.

It will be appreciated by those of ordinary skill in the art that the method of amplification disclosed herein, by which a heterologous DNA is replicated under the control of a bacteriophage phi29 replication origin, has applications in many different aspects of molecular biology, and also in the diagnostic arts, and is not limited to use in conjunction with the methods of cloning and mapping genomic DNA of the present invention. For example, by joining a bacteriophage phi29 replication origin to one end of a double-stranded DNA, a means is provided for synthesizing a multiplicity of single-stranded DNAs suitable for such uses as templates for DNA sequencing or as hybridization probes.

If in an amplification step two or more different genomic DNA restriction fragments are amplified in the same reaction, it will be necessary to separate the different restriction fragments from one another in order to produce individual genomic DNA clones. Separation of the different restriction fragments may be accomplished subsequent to amplification by any of the known methods for physically separating nucleic acids, such as chromatography, centrifugation, or electrophoresis. Lengthy DNA molecules, in the size range from about 10,000 base pairs to 100,000 base pairs or more are preferably separated by the technique of pulsed field gel electrophoresis. Carle and Olson, 1984, Nuc. Acids. Res. 12:5647-5664; Smith, et al., 1986, Genetic Engineering 8:45-70 (Plenum Press, New York). The separated DNA molecules can then be isolated in any known manner and, if desired, the isolated DNA molecules again may be amplified to produce a multiplicity of copies of each individual genomic DNA clone.

Alternatively, the different DNA restriction fragments may be labeled with moieties capable of producing, either directly or indirectly, different detectable signals, as for example by incorporating such moieties in the different linkers that are joined with the DNA restriction fragments, and physically separating the DNA restriction fragments on the basis of those different signals. Thus, for example, the detectable moiety can be a fluorophore, and the DNA restriction fragments separated by flow cytometry. Gray, et al., 1987, Science 238:323-329.

In yet a further aspect, the present invention provides efficient means for the physical mapping of genomic DNA restriction fragments having multiple different cohesive-end sequences. After digesting genomic DNA with a restriction enzyme capable of generating multiple different cohesive-end sequences, the relative positions of the resulting restriction fragments within the substrate genomic DNA are established by determining the sequence at one or both ends of each different restriction fragment, including at least the sequence of each cohesive-end. Provided the cohesive-end sequences differ between each different restriction fragment, alignment of the restriction fragments is conveniently accomplished by taking advantage of the fact that restriction fragments that are adjacent within the genomic DNA substrate, and therefore share common cleavage sites, will have complementary cohesive-end sequences.

The nucleotide sequence at the end of a genomic DNA restriction fragment may be determined directly by any of the known methods of nucleic acid sequencing, for example, the chemical degradation technique of Maxam and Gilbert, 1980, Meth. Enzymol. 65:499-560 or the chain termination technique of Sanger, et al., 1977, Proc. Nat. Acad. Sci. 74:5463-5467, or may be determined indirectly from the cohesive-end sequence of a linker(s) that is joined to the restriction fragment.

The manipulation and analysis of accumulated sequence data, particularly the aligning of individual genomic DNA restriction fragments in a physical map, is conveniently accomplished using a computer method. For example, Staden, 1982, Nuc. Acids Res. 10:4731-4751, describes a computer method for handling DNA sequence information, and aligning DNA restriction fragment clones that are related to one another by overlap of their sequences.

The following examples are offered by way of illustration, and are not intended to limit the invention in any manner. All patent and literature references described herein are expressly incorporated.

## Examples

### Digestion of Adenovirus-2 DNA with *Sfi*I

Purified adenovirus-2 DNA (International Biotechnologies, Inc., New Haven, CT) is digested with restriction endonuclease *Sfi*I (New England BioLabs, Beverly, MA) in a reaction consisting of 10mM Tris-HCl (pH 7.9), 10mM $MgCl_2$, 10mM 2-mercaptoethanol, 50mM NaCl, 100μg/ml bovine serum albumin, 20μg/ml adenovirus-2 DNA, and 5 units *Sfi* I in a total reaction volume of 100 μl, for 1 hour at 50 °C.

Removal of Adenovirus-2 Terminal Protein

Adenovirus-2 DNA contains a virus-encoded protein covalently bound to the 5' terminus of each strand of the linear DNA molecule, removal of which is necessary to produce ends capable of ligation with linkers. Stillman, et al., 1981, Cell 23:497-508; Tamanoi, et al., 1982, Proc. Nat. Acad. Sci. 79:2221-2225. To separate the 5' terminal protein from adenovirus-2 DNA, 20 µg/ml adenovirus-2 DNA in 10mm Tris-HCl (pH 7.5) is mixed with an equal volume of 1M piperidine, incubated for 2 hours at 37°C, then lyophilized, dissolved in water and lyophilized again.

Oligonucleotide Synthesis

The single-stranded 12-mer oligonucleotide 5' GGGGCTTACTTT 3' and each of 64 different single-stranded 15-mer oligonucleotides having the general sequence 5' AAAGTAAGCCCCNNN 3', wherein N is any of the four nucleotides adenine, thymine, guanine, and cytosine, are synthesized on a Biosearch Model 8600 DNA Synthesizer in accordance with the method of Froehler, et al., supra. To prepare linkers, each of the different 15-mer oligonucleotides is annealed with the complementary 12-mer oligonucleotide in hybridization buffer, consisting of 10mM Tris-HCl (pH 7.5), 10mM $MgCl_2$, 20mM NaCl, at a temperature between 25° and 30°C. The nucleotide sequences of the 64 linker products, having the general structure

5' AAAGTAAGCCCCNNN 3'
3' TTTCATTCGGGG 5'

are shown in Fig. 1.

Cloning of the Adenovirus-2 Genome

Adenovirus-2 terminal protein is removed from the ends adenovirus-2 DNA as described above. Linkers comprising two bacteriophage phi29 left origins of replication in a tail-to-tail orientation, and having the sequence

5' AAAGTAAGCCCCGGGGCTTACTTT 3'
3' TTTCATTCGGGGCCCCGAATGAAA 5'

are then joined to the resulting blunt ends of the adenovirus-2 DNA, using 100 pmoles of linker per microgram of adenovirus-2 DNA in ligation buffer consisting of 50mM Tris-HCl (pH 7.8), 10mM $MgCl_2$, 2mM dithiothreitol, 1mM ATP, 1mM spermidine, and 50 µg/ml bovine serum albumin. 2 units of T4 DNA ligase (New England BioLabs, Beverly, MA) are then added per microgram of adenovirus-2 in the reaction mixture, and the reaction mixture is then incubated at 15°C for one hour.

Following ligation of the bacteriophage phi29 origin linkers to the original ends of the adenovirus-2 DNA, the adenovirus-2 DNA is digested with *Sfi* I as described above. The cloning of the resulting *Sfi* I restriction fragments is then conveniently carried out according to the methods of the invention in the individual wells of 96-well microtiter plates.

To each separate well of the microtiter plates is added 100 pmoles each of two different linkers of Fig. 1, such that every possible combination of two different linkers of Fig. 1 is contained in one or another microtiter well. The number of different combinations of the 64 linkers of Fig. 1, and thus the number of microtiter wells that need be prepared for the cloning of *Sfi* I restriction fragments, is 2016. In general, the number of different combinations of N different linkers may be determined according to the formula:

$$\text{number of combinations} = \frac{N^2 - N}{2}.$$

1 µg of *Sfi* I digested adenovirus-2 DNA is then added to each separate microtiter well in 25 µl of ligation buffer together with 2 units of T4 DNA ligase (New England BioLabs, Beverly, MA). The reaction mixture is incubated at 15°C for one hour to allow the ligation of linkers to the cohesive-ends of the adenovirus-2 DNA restriction fragments. The ligation reaction is stopped by heating for 15 minutes at 68°C.

Following ligation of linkers to the adenovirus-2 DNA restriction fragments, amplification of the adenovirus-2 restriction fragments is accomplished by adding to the reaction mixture in each microtiter well 20μM each dATP, dTTP, dGTP, and dCTP, 20mM $(NH_4)_2SO_4$, 5% (vol/vol) glycerol, 300 ng of purified bacteriophage phi29 terminal protein, and 20 ng of bacteriophage phi29 DNA polymerase. After incubation for 2 hours at 30 °C., the DNA synthesis reaction is stopped by the addition of 10mM EDTA and heating for 10 minutes at 68 °C.

Following polyacrylamide gel electrophoresis of the DNA synthesis reaction mixtures, each of the amplified adenovirus-2 DNA restriction fragments is visualized by staining the gel with ethidium bromide, and then purified from the gel by electroelution. Sequencing of the ends of each strand of the restriction fragments is carried out according to the method of Maxam and Gilbert, supra, using $\alpha^{32}$P-cordycepin-5'-triphosphate for 3'-end labeling. Tu, et al., 1980, Gene 10:177-183.

Physical Mapping of the Adenovirus-2 Genome

Digestion of adenovirus-2 DNA with *Sfi* I generates four restriction fragments, ranging in size from approximately 1000 base pairs to approximately 16,000 base pairs. The nucleotide sequence at the 3' ends of the each of the restriction fragments is as follows:

```
Fragment        Sequence
   1      5'            . . . . . . . TGC 3'
          3'  GTA       . . . . . . .     5'
   2      5'            . . . . . . . GAC 3'
          3'  GAC       . . . . . . .     5'
   3      5'            . . . . . ATG 3'
          3'  CTG       . . . .       5'
   4      5'            . . . . . CTG 3'
          3'  ACG       . . . .       5'
```

Alignment of the restriction fragments in a physical map is accomplished by analyzing the nucleotide sequence of each restriction fragment for complementary 3'-end sequences. Accordingly, the four *Sfi* I restriction fragments of adenovirus-2 DNA are aligned as follows:

```
5'       . . . . TGC 3'
3' GTA . . . .   |||  5'
   (fragment 1)  |||
                 |||
             5'  ||| . . . . CTG 3'
             3'  ACG . . . .  |||  5'
                 (fragment 4) |||
                              |||
                          5'  ||| . . . . GAC 3'
                          3'  GAC . . . .  |||  5'
                              (fragment 2) |||
                                           |||
                                       5'  ||| . . . . ATG 3'
                                       3'  CTG . . . .     5'
                                           (fragment 3)
```

Cloning of an Internal *Sfi* I Fragment of Adenovirus-2 DNA

This example illustrates an embodiment of the invention wherein a genomic DNA restriction fragment having a predetermined cohesive-end sequence is cloned.

Adenovirus-2 DNA is digested with *Sfi* I as described above. From the known nucleotide sequence of the adenovirus-2 genome, Roberts, et al., 1986, in "Adenovirus DNA," W. Doerfler (ed.) (Martinus Nijhoff

Publishing, Boston, MA), it may be predicted that one of the resulting restriction fragments, extending from nucleotide 17305 to nucleotide 23046 of the adenovirus-2 genome, will have the following structure:

5' CGGC . . . GCCGGAC 3'
3' GACGCCG . . . CGGC 5'.

As the first step in the in vitro cloning of this restriction fragment, 1 μg of *Sfi* I digested adenovirus-2 DNA is mixed with 100 pmoles of oligonucleotide linker having the sequence

5' AAAGTAAGCCCCCTG 3'
3' TTTCATTCGGGG 5'

and 100 pmoles of oligonucleotide linker having the sequence

5' AAAGTAAGCCCCGTC 3'
3' TTTCATTCGGGG 5'

in 25 μl of ligation buffer. 2 units of T4 DNA ligase are then added to the mixture and the ligation reaction allowed to proceed for one hour at 15° C.

Following ligation of the linkers to the complementary cohesive-ends of the desired adenovirus-2 DNA restriction fragment, amplification of the restriction fragment is accomplished by adding to the ligation reaction mixture 20μM each dATP, dTTP, dGTP, and dCTP, 20mM $(NH_4)_2SO_4$, 5% (vol/vol) glycerol, 300 ng of purified bacteriophage phi29 terminal protein, and 20 ng of bacteriophage phi29 DNA polymerase. After incubation for 20 minutes at 30° C., the DNA synthesis reaction is stopped by the addition of 10mM EDTA and heating for 10 minutes at 68° C.

## Claims

**1.** A method for amplification of a nucleic acid sequence heterologous to bacteriophage phi29 which comprises:
(a) synthesizing a double-stranded nucleic acid which includes the heterologous sequence and a bacteriophage phi29 replication origin; and
(b) synthesizing DNA from said double-stranded nucleic acid under the control of said replication origin.

**2.** The method of claim 1 wherein the double-stranded nucleic acid of step (a) is obtained by ligation of a bacteriophage phi29 replication origin to a double-stranded nucleic acid.

**3.** The method of claim 1 wherein the double-stranded nucleic acid of step (a) is obtained by steps comprising:
(1) providing an oligonucleotide origin-primer comprising a bacteriophage phi29 replication origin joined to a primer, and an oligonucleotide secondary primer, wherein said primers are not complementary to one another, and are selected such that the extension product synthesized from one primer, when separated from its complement, can serve as a template for synthesis of the extension product of the other primer;
(2) contacting a nucleic acid under hybridizing conditions with the origin-primer such that an origin-primer extension product is synthesized; and
(3) contacting a nucleic acid under hybridizing conditions with the secondary primer such that a secondary primer extension product is synthesized.

**4.** The method of claim 1 wherein replication originates at one end of said double-stranded nucleic acid and the DNA products of step (b) are single-stranded.

**5.** The method of claim 1 wherein replication originates at both ends of said double-stranded nucleic acid and the DNA products of step (b) are double-stranded.

**6.** A method for amplification of a nucleic acid sequence heterologous to bacteriophage phi29 which comprises:
(a) providing a double-stranded nucleic acid which includes the heterologous sequence;
(b) providing a DNA linker which includes a bacteriophage phi29 replication origin;
(c) joining said linker to said double-stranded nucleic acid; and
(d) contacting the product of step (c) with bacteriophage phi29 DNA polymerase, bacteriophage phi29 terminal protein, and four different deoxynucleoside triphosphates under conditions such that DNA is synthesized.

**7.** The method of claims 1 or 6 wherein the bacteriophage phi29 replication origin comprises the sequence 5' AAAGTAAGCCCC 3'.

**8.** The method of claims 1 or 6 wherein the bacteriophage phi29 replication origin comprises the sequence 5' AAAGTAGGGTAC 3'.

**9.** The method of claims 1 or 6 wherein the heterologous nucleic acid sequence is eukaryotic.

**10.** The method of claims 1 or 6 wherein the heterologous nucleic acid sequence is human.

**11.** The method of claim 6 wherein the DNA linker of step (b) is prepared by in vitro synthesis.

**12.** A replicable DNA comprising a bacteriophage phi29 replication origin at one or both ends of a DNA sequence heterologous to bacteriophage phi29.

**13.** The product of claim 12 wherein the heterologous DNA sequence is eukaryotic.

**14.** The product of claim 12 wherein the heterologous DNA sequence is human.

**15.** An origin-primer comprising a bacteriophage phi29 replication origin joined to the 5' end of a primer.

**16.** A method for cloning genomic DNA which comprises:
(a) digesting genomic DNA with a restriction enzyme that generates multiple different cohesive-end sequences;
(b) contacting the digested genomic DNA with a linker, said linker comprising a reporter nucleotide sequence comprising a bacteriophage phi29 replication origin and a cohesive-end sequence complementary to a cohesive-end sequence generated by the restriction enzyme of step (a), under conditions such that a linker is joined to a restriction fragment of genomic DNA; and
(c) amplifying the product of step (b) using bacteriophage phi29 DNA polymerase.

**17.** The method of claim 16 wherein the digested genomic DNA is contacted with two or more different linkers in step (b).

**18.** The method of claim 16 wherein the cohesive-end sequences generated by the restriction enzyme of step (a) are of the same length.

**19.** The method of claim 16 wherein the restriction enzyme of step (a) is S*fi*I.

**20.** The method of claims 16 or 17 wherein steps (b) and (c) are repeated at least once.

**21.** The method of claims 16 or 17 wherein the product of step (b) is separated from the digested genomic DNA before or after step (c).

**22.** The method of claim 21 wherein the product of step (b) is separated by eletrophoresis.

**23.** A method for mapping genomic DNA restriction fragments which comprises:
(a) digesting genomic DNA with a restriction enzyme that generates multiple different cohesive-end sequences;
(b) amplifying the restriction fragment products of step (a) by

(1) providing a library of linkers, wherein:

each linker comprises a bacteriophage phi29 replication origin and a cohesive-end sequence, and

the library comprises linkers having cohesive-end sequences complementary to substantially every cohesive-end sequence generated by the restriction enzyme of step (a);

(2) contacting the restriction fragments of step (a) with linkers from said library under conditions such that each restriction fragment cohesive-end is joined with a linker; and

(3) contacting the products of step (2) with bateriophage phi29 DNA polymerase, bacteriophage phi29 DNA synthesis priming protein, and four different nucleoside triphosphates under conditions such that multiple copies of each restriction fragment are synthesized under the control of said bacteriophage phi29 replication origin;

(c) determining the cohesive-end sequence of each of said restriction fragments, and in accordance therewith,

(d) aligning the restriction fragments in a physical map.

**24.** The method of claim 23 wherein the method further comprises separating the restriction fragment products of step (a) from one another before or after step (b).

**25.** The method of claim 23 wherein the method further comprises separating the restriction fragment products of step (a) from one another by gel eletrophoresis before or after step (b).

**26.** The method of claim 23 wherein step (2) further comprises contacting the restriction fragment products of step (a) with several different combinations of linkers in separate reaction vessels, each combination of linkers comprising two or more linkers from said library.

**27.** The method of claim 23 wherein the genomic DNA of step (a) is eukaryotic.

**28.** The method of claim 23 wherein the genomic DNA of step (a) is human.

**29.** The method of claim 23 wherein the restriction enzyme of step (a) is S*fi*I.

**Patentansprüche**

**1.** Verfahren zur Amplifikation einer zum Bakteriophagen phi29 heterologen Nukleinsäuresequenz, welches umfaßt:

(a) das Synthetisieren einer doppelstrangigen Nukleinsäure, die die heterologe Sequenz und einen Bakteriophage phi29-Replikationsursprung enthält; und

(b) das Synthetisieren von DNA aus der genannten doppelstrangigen Nukleinsäure unter der Steuerung des genannten Replikationsursprunges.

**2.** Verfahren nach Anspruch 1, worin die doppelstrangige Nukleinsäure von Schritt (a) durch Ligation eines Bakteriophage phi29-Replikationsursprungs an eine doppelstrangige Nukleinsäure erhalten wird.

**3.** Verfahren nach Anspruch 1, worin die doppelstrangige Nukleinsäure von Schritt (a) durch Schritte erhalten wird, die umfassen:

(1) das Vorsehen eines Oligonukleotidursprung-Primers, der einen an einen Primer angefügten Bakteriophage phi29-Replikationsursprung umfaßt, und eines sekundären Oligonukleotidprimers, worin die genannten Primer zueinander nicht komplementär und so ausgewählt sind, daß das von einem Primer synthetisierte Extensionsprodukt, wenn es von seinem Komplement getrennt ist, als eine Schablone zur Synthese des Extensionsproduktes des anderen Primers dienen kann;

(2) das In-Kontakt-Bringen einer Nukleinsäure unter hybridisierenden Bedingungen mit dem Ursprungsprimer, sodaß ein Ursprungsprimer-Extensionsprodukt synthetisiert werden kann; und

(3) das In-Kontakt-Bringen einer Nukleinsäure unter hybridisierenden Bedingungen mit dem sekundären Primer, sodaß ein sekundäres Primerextensionsprodukt synthetisiert wird.

**4.** Verfahren nach Anspruch 1, worin die Replikation an einem Ende der genannten doppelstrangigen Nukleinsäure ihren Ausgang nimmt und die DNA-Produkte von Schritt (b) einstrangig sind.

5. Verfahren nach Anspruch 1, worin die Replikation an beiden Enden der genannten doppelstrangigen Nukleinsäure ihren Ausgang nimmt und die DNA-Produkte von Schritt (b) doppelstrangig sind.

6. Verfahren zur Amplifikation einer zu Bakteriophage phi29 heterologen Nukleinsäuresequenz, welches umfaßt:
   (a) das Vorsehen einer doppelstrangigen Nukleinsäure, die die heterologe Sequenz enthält;
   (b) das Vorsehen eines DNA-Linkers, der einen Bakteriophage phi29-Replikationsursprung enthält;
   (c) das Anfügen des genannten Linkers an die genannte doppelstrangige Nukleinsäure; und
   (d) das In-Kontakt-Bringen des Produktes von Schritt (c) mit Bakteriophage phi29-DNA-Polymerase, Bakteriophage phi29-Terminalprotein und vier verschiedenen Desoxynukleosidtriphosphaten unter Bedingungen, sodaß DNA synthetisiert wird.

7. Verfahren nach Anspruch 1 oder 6, worin der Bakteriophage phi29-Replikationsursprung die Sequenz 5' AAAGTAAGCCCC 3' umfaßt.

8. Verfahren nach Anspruch 1 oder 6, worin der Bakteriophage phi 29-Replikationsursprung die Sequenz 5' AAAGTAGGGTAC 3' umfaßt.

9. Verfahren nach Anspruch 1 oder 6, worin die heterologe Nukleinsäuresequenz eukaryotisch ist.

10. Verfahren nach Anspruch 1 oder 6, worin die heterologe Nukleinsäuresequenz vom Menschen stammt.

11. Verfahren nach Anspruch 6, worin der DNA-Linker von Schritt (b) durch Synthese in vitro hergestellt wird.

12. Replizierbare DNA, die einen Bakteriophage phi29-Replikationsursprung an einem oder beiden Enden einer DNA-Sequenz umfaßt, die zum Bakteriophagen phi 29 heterolog ist.

13. Produkt nach Anspruch 12, worin die heterologe DNA-Sequenz eukaryotisch ist.

14. Produkt nach Anspruch 12, worin die heterologe DNA-Sequenz vom Menschen stammt.

15. Ursprungsprimer, umfassend einen an das 5'-Ende eines Primers angefügten Bakteriophage phi29 Replikationsursprung.

16. Verfahren zum Klonieren genomischer DNA, welches umfaßt:
   (a) das Digerieren von genomischer DNA mit einem Restriktionsenzym, das multiple unterschiedliche Sequenzen mit kohäsivem Ende erzeugt;
   (b) das In-Kontakt-Bringen der digerierten genomischen DNA mit einem Linker, wobei der genannte Linker eine Reporter-Nukleotidsequenz umfaßt, die einen Bakteriophage phi29-Replikationsursprung und eine Sequenz mit kohäsivem Ende umfaßt, die zu einer Sequenz mit kohäsivem Ende komplementär ist, die durch das Restriktionsenzym von Schritt (a) erzeugt wird, unter Bedingungen, sodaß ein Linker an ein Restriktionsfragment von genomischer DNA angefügt ist; und
   (c) das Verstärken des Produktes von Schritt (b) unter Verwendung von Bakteriophage phi29-DNA-Polymerase.

17. Verfahren nach Anspruch 16, worin die digerierte genomische DNA in Schritt (b) mit zwei oder mehr verschiedenen Linkern in Kontakt gebracht wird.

18. Verfahren nach Anspruch 16, worin die durch das Restriktionsenzym von Schritt (a) erzeugten Sequenzen mit kohäsiven Enden die gleiche Länge haben.

19. Verfahren nach Anspruch 16, worin das Restriktionsenzym von Schritt (a) SfiI ist.

20. Verfahren nach Anspruch 16 oder 17, worin die Schritte (b) und (c) zumindest einmal wiederholt werden.

**21.** Verfahren nach Anspruch 16 oder 17, worin das Produkt von Schritt (b) vor oder nach Schritt (c) von der digerierten genomischen DNA getrennt wird.

**22.** Verfahren nach Anspruch 21, worin das Produkt von Schritt (b) durch Elektrophorese abgetrennt wird.

**23.** Verfahren zum Mappen von genomischen DNA-Restriktionsfragmenten, welches umfaßt:
(a) das Digerieren von genomischer DNA mit einem Restriktionsenzym, das multiple unterschiedliche Sequenzen mit kohäsiven Enden erzeugt;
(b) die Amplifikation der Restriktionsfragmentprodukte von Schritt (a) durch
(1) das Vorsehen einer Sammlung von Linkern, worin:
jeder Linker einen Bakteriophage phi29-Replikationsursprung und eine Sequenz mit kohäsivem Ende umfaßt, und
die Sammlung Linker umfaßt, die Sequenzen mit kohäsiven Enden aufweisen, die zu im wesentlichen jeder Sequenz mit kohäsivem Ende komplementär sind, die durch das Restriktionsenzym von Schritt (a) erzeugt werden;
(2) das In-Kontakt-Bringen der Restriktionsfragmente von Schritt (a) mit Linkern von der genannten Sammlung unter Bedingungen, sodaß jedes kohäsive Ende des Restriktionsfragments an einen Linker angefügt ist; und
(3) das In-Kontakt-Bringen der Produkte von Schritt (2) mit Bakteriophage phi29-DNA-Polymerase, Bakteriophage phi29-DNA-Synthese-Primerprotein und vier verschiedenen Nukleosidtriphosphaten unter Bedingungen, sodaß mehrere Kopien eines jeden Restriktionsfragmentes gesteuert vom genannten Bakteriophage phi29-Replikationsursprung synthetisiert werden;
(c) das Ermitteln der Sequenz mit kohäsivem Ende eines jeden der genannten Restriktionsfragmente, und dementsprechend
(d) das Ausrichten der Restriktionsfragmente in einem physischen Plan.

**24.** Verfahren nach Anspruch 23, worin das Verfahren weiters das Abtrennen der Restriktionsfragmentprodukte von Schritt (a) voneinander vor oder nach Schritt (b) umfaßt.

**25.** Verfahren nach Anspruch 23, worin das Verfahren weiters das Abtrennen der Restriktionsfragmentprodukte von Schritt (a) voneinander durch Gelelektrophorese vor oder nach Schritt (b) umfaßt.

**26.** Verfahren nach Anspruch 23, worin Schritt (2) weiters das In-Kontakt-Bringen der Restriktionsfragmentprodukte von Schritt (a) mit mehreren unterschiedlichen Kombinationen von Linkern in getrennten Reaktionsgefäßen umfaßt, wobei jede Linkerkombination zwei oder mehr Linker von der genannten Sammlung umfaßt.

**27.** Verfahren nach Anspruch 23, worin die genomische DNA von Schritt (a) eukaryotisch ist.

**28.** Verfahren nach Anspruch 23, worin die genomische DNA von Schritt (a) vom Menschen stammt.

**29.** Verfahren nach Anspruch 23, worin das Restriktionsenzym von Schritt (a) SfiI ist.

**Revendications**

**1.** Procédé d'amplification d'une séquence d'acide nucléique hétérologue vis-à-vis du bactériophage phi29, qui comprend :
(a) la synthèse d'un acide nucléique bicaténaire qui comprend la séquence hétérologue et une origine de réplication du bactériophage phi29 ; et
(b) la synthèse d'un ADN à partir dudit acide nucléique bicaténaire sous le contrôle de ladite origine de réplication.

**2.** Procédé suivant la revendication 1, dans lequel l'acide nucléique bicaténaire de l'étape (a) est obtenu par ligation d'une origine de réplication de bactériophage phi29 à un acide nucléique bicaténaire.

**3.** Procédé suivant la revendication 1, dans lequel l'acide nucléique bicaténaire de l'étape (a) est obtenu par des étapes consistant :

(1) à prendre une origine-amorce oligonucléotidique comprenant une origine de réplication de bactériophage phi29 jointe à une amorce, et une amorce secondaire oligonucléotidique, dans laquelle lesdites amorces ne sont pas mutuellement complémentaires et sont choisies de telle sorte que le produit d'extension synthétisé à partir d'une amorce, lorsqu'il est séparé de son complément, puisse servir de matrice pour la synthèse du produit d'extension de l'autre amorce ;
(2) à mettre en contact un acide nucléique dans des conditions d'hybridation avec l'origine-amorce de telle sorte qu'un produit d'extension d'origine-amorce soit synthétisé ; et
(3) à mettre en contact un acide nucléique dans des conditions d'hybridation avec l'amorce secondaire de telle sorte qu'un produit d'extension d'amorce secondaire soit synthétisé.

**4.** Procédé suivant la revendication 1, dans lequel la réplication débute à une extrémité de l'acide nucléique bicaténaire et les produits de l'étape (b) consistant en ADN sont monocaténaires.

**5.** Procédé suivant la revendication 1, dans lequel la réplication débute aux deux extrémités de l'acide nucléique bicaténaire et les produits de l'étape (b) consistant en ADN sont bicaténaires.

**6.** Procédé d'amplification d'une séquence d'acide nucléique hétérologue vis-à-vis du bactériophage phi29, qui comprend les étapes consistant :
(a) à prendre un acide nucléique bicaténaire qui comprend la séquence hétérologue ;
(b) à prendre un segment de liaison d'ADN qui comprend une origine de réplication de bactériophage phi29 ;
(c) à joindre ledit segment de liaison audit acide nucléique bicaténaire ; et
(d) à mettre en contact le produit de l'étape (c) avec l'ADN-polymérase de bactériophage phi29, la protéine terminale de bactériophage phi29 et quatre désoxynucléosidetriphosphates différents dans des conditions telles qu'un ADN soit synthétisé.

**7.** Procédé suivant la revendication 1 ou 6, dans lequel l'origine de réplication du bactériophage phi29 comprend la séquence 5'AAAGTAAGCCCC3'.

**8.** Procédé suivant la revendication 1 ou 6, dans lequel l'origine de réplication du bactériophage phi29 comprend la séquence 5'AAAGTAGGGTAC3'.

**9.** Procédé suivant la revendication 1 ou 6, dans lequel la séquence d'acide nucléique hétérologue est eucaryotique.

**10.** Procédé suivant la revendication 1 ou 6, dans lequel la séquence d'acide nucléique hétérologue est d'origine humaine.

**11.** Procédé suivant la revendication 6, dans lequel le segment de liaison d'ADN de l'étape (b) est préparé par synthèse in vitro.

**12.** ADN réplicable comprenant une origine de réplication de bactériophage phi29 à une extrémité ou aux deux extrémités d'une séquence d'ADN hétérologue vis-à-vis du bactériophage phi29.

**13.** Produit suivant la revendication 12, dans lequel la séquence d'ADN hétérologue est eucaryotique.

**14.** Produit suivant la revendication 12, dans lequel la séquence d'ADN hétérologue est d'origine humaine.

**15.** Origine-amorce comprenant une origine de réplication de bactériophage phi29 jointe à l'extrémité 5' d'une amorce.

**16.** Procédé de clonage d'ADN génomique, qui comprend :
(a) la digestion d'un ADN génomique avec un enzyme de restriction qui engendre des séquences à extrémités cohésives différentes multiples ;
(b) la mise en contact de l'ADN génomique digéré avec un segment de liaison, ledit segment de liaison comprenant une séquence de nucléotides de rapporteur comprenant une origine de réplication de bactériophage phi29 et une séquence à extrémités cohésives complémentaire d'une séquence à extrémités cohésives engendrée par l'enzyme de restriction de l'étape (a), dans des

conditions telles qu'un segment de liaison soit joint à un fragment de restriction d'ADN génomique ; et
(c) l'amplification du produit de l'étape (b) au moyen de l'ADN-polymérase de bactériophage phi29.

**17.** Procédé suivant la revendication 16, dans lequel l'ADN génomique digéré est mis en contact avec deux ou plus de deux segments de liaison différents dans l'étape (b).

**18.** Procédé suivant la revendication 16, dans lequel les séquences à extrémités cohésives engendrées par l'enzyme de restriction de l'étape (a) possèdent la même longueur.

**19.** Procédé suivant la revendication 16, dans lequel l'enzyme de restriction de l'étape (a) est l'enzyme SfiI.

**20.** Procédé suivant la revendication 16 ou 17, dans lequel les étapes (b) et (c) sont répétées au moins une fois.

**21.** Procédé suivant la revendication 16 ou 17, dans lequel le produit de l'étape (b) est séparé de l'ADN génomique digéré avant ou après l'étape (c).

**22.** Procédé suivant la revendication 21, dans lequel le produit de l'étape (b) est séparé par électrophorèse.

**23.** Procédé de cartographie de fragments de restriction d'ADN génomique, qui comprend :
(a) la digestion d'un ADN génomique avec un enzyme de restriction qui engendre des séquences à extrémités cohésives différentes multiples ;
(b) l'amplification des fragments de restriction formés dans l'étape (a)
(1) en utilisant une banque de segments de liaison, dans laquelle :
chaque segment de liaison comprend une origine de réplication de bactériophage phi29 et une séquence à extrémités cohésives, et
la banque comprend des segments de liaison possédant des séquences à extrémités cohésives complémentaires de pratiquement chaque séquence à extrémités cohésives engendrée par l'enzyme de restriction de l'étape (a) ;
(2) en mettant en contact les fragments de restriction de l'étape (a) avec des segments de liaison provenant de ladite banque dans des conditions telles que chaque extrémité cohésive de fragment de restriction soit jointe à un segment de liaison ; et
(3) en mettant en contact les produits de l'étape (2) avec l'ADN-polymérase de bactériophage phi29, la protéine d'amorçage de synthèse d'ADN du bactériophage phi29 et quatre nucléoside-triphosphates différents dans des conditions telles que des copies multiples de chaque fragment de restriction soient synthétisées sous le contrôle de ladite origine de réplication du bactériophage phi29 ;
(c) la détermination de la séquence à extrémités cohésives de chacun desdits fragments de restriction et, en fonction de cette séquence,
(d) l'alignement des fragments de restriction dans une carte physique.

**24.** Procédé suivant la revendication 23, qui comprend en outre la séparation des fragments de restriction formés dans l'étape (a) les uns des autres avant ou après l'étape (b).

**25.** Procédé suivant la revendication 23, qui comprend en outre la séparation des fragments de restriction formés dans l'étape (a) les uns des autres par électrophorèse sur gel avant ou après l'étape (b).

**26.** Procédé suivant la revendication 23, dans lequel l'étape (2) comprend en outre la mise en contact des fragments de restriction formés dans l'étape (a) avec plusieurs associations différentes de segments de liaison dans des récipients réactionnels distincts, chaque association de segments de liaison comprenant deux ou plus de deux segments de liaison provenant de la banque.

**27.** Procédé suivant la revendication 23, dans lequel l'ADN génomique de l'étape (a) est eucaryotique.

**28.** Procédé suivant la revendication 23, dans lequel l'ADN génomique de l'étape (a) est d'origine humaine.

**29.** Procédé suivant la revendication 23, dans lequel l'enzyme de restriction de l'étape (a) est l'enzyme SfiI.

FIGURE 1a

| | |
|---|---|
| 5' AAAGTAAGCCCCAAA 3'<br>3' TTTCATTCGGGG 5' | 5' AAAGTAAGCCCCAAT 3'<br>3' TTTCATTCGGGG 5' |
| 5' AAAGTAAGCCCCAAG 3'<br>3' TTTCATTCGGGG 5' | 5' AAAGTAAGCCCCAAC 3'<br>3' TTTCATTCGGGG 5' |
| 5' AAAGTAAGCCCCATA 3'<br>3' TTTCATTCGGGG 5' | 5' AAAGTAAGCCCCATT 3'<br>3' TTTCATTCGGGG 5' |
| 5' AAAGTAAGCCCCATG 3'<br>3' TTTCATTCGGGG 5' | 5' AAAGTAAGCCCCATC 3'<br>3' TTTCATTCGGGG 5' |
| 5' AAAGTAAGCCCCAGA 3'<br>3' TTTCATTCGGGG 5' | 5' AAAGTAAGCCCCAGT 3'<br>3' TTTCATTCGGGG 5' |
| 5' AAAGTAAGCCCCAGG 3'<br>3' TTTCATTCGGGG 5' | 5' AAAGTAAGCCCCAGC 3'<br>3' TTTCATTCGGGG 5' |
| 5' AAAGTAAGCCCCACA 3'<br>3' TTTCATTCGGGG 5' | 5' AAAGTAAGCCCCACT 3'<br>3' TTTCATTCGGGG 5' |
| 5' AAAGTAAGCCCCACG 3'<br>3' TTTCATTCGGGG 5' | 5' AAAGTAAGCCCCACC 3'<br>3' TTTCATTCGGGG 5' |
| 5' AAAGTAAGCCCCTAA 3'<br>3' TTTCATTCGGGG 5' | 5' AAAGTAAGCCCCTAT 3'<br>3' TTTCATTCGGGG 5' |
| 5' AAAGTAAGCCCCTAG 3'<br>3' TTTCATTCGGGG 5' | 5' AAAGTAAGCCCCTAC 3'<br>3' TTTCATTCGGGG 5' |
| 5' AAAGTAAGCCCCTTA 3'<br>3' TTTCATTCGGGG 5' | 5' AAAGTAAGCCCCTTT 3'<br>3' TTTCATTCGGGG 5' |
| 5' AAAGTAAGCCCCTTG 3'<br>3' TTTCATTCGGGG 5' | 5' AAAGTAAGCCCCTTC 3'<br>3' TTTCATTCGGGG 5' |
| 5' AAAGTAAGCCCCTGA 3'<br>3' TTTCATTCGGGG 5' | 5' AAAGTAAGCCCCTGT 3'<br>3' TTTCATTCGGGG 5' |
| 5' AAAGTAAGCCCCTGG 3'<br>3' TTTCATTCGGGG 5' | 5' AAAGTAAGCCCCTGC 3'<br>3' TTTCATTCGGGG 5' |
| 5' AAAGTAAGCCCCTCA 3'<br>3' TTTCATTCGGGG 5' | 5' AAAGTAAGCCCCTCT 3'<br>3' TTTCATTCGGGG 5' |
| 5' AAAGTAAGCCCCTCG 3'<br>3' TTTCATTCGGGG 5' | 5' AAAGTAAGCCCCTCC 3'<br>3' TTTCATTCGGGG 5' |

FIGURE 1b

5' AAAGTAAGCCCCGAA 3'
3' TTTCATTCGGGG 5'

5' AAAGTAAGCCCCGAG 3'
3' TTTCATTCGGGG 5'

5' AAAGTAAGCCCCGTA 3'
3' TTTCATTCGGGG 5'

5' AAAGTAAGCCCCGTG 3'
3' TTTCATTCGGGG 5'

5' AAAGTAAGCCCCGGA 3'
3' TTTCATTCGGGG 5'

5' AAAGTAAGCCCCGGG 3'
3' TTTCATTCGGGG 5'

5' AAAGTAAGCCCCGCA 3'
3' TTTCATTCGGGG 5'

5' AAAGTAAGCCCCGCG 3'
3' TTTCATTCGGGG 5'

5' AAAGTAAGCCCCCAA 3'
3' TTTCATTCGGGG 5'

5' AAAGTAAGCCCCCAG 3'
3' TTTCATTCGGGG 5'

5' AAAGTAAGCCCCCTA 3'
3' TTTCATTCGGGG 5'

5' AAAGTAAGCCCCCTG 3'
3' TTTCATTCGGGG 5'

5' AAAGTAAGCCCCCGA 3'
3' TTTCATTCGGGG 5'

5' AAAGTAAGCCCCCGG 3'
3' TTTCATTCGGGG 5'

5' AAAGTAAGCCCCCCA 3'
3' TTTCATTCGGGG 5'

5' AAAGTAAGCCCCCCG 3'
3' TTTCATTCGGGG 5'

5' AAAGTAAGCCCCGAT 3'
3' TTTCATTCGGGG 5'

5' AAAGTAAGCCCCGAC 3'
3' TTTCATTCGGGG 5'

5' AAAGTAAGCCCCGTT 3'
3' TTTCATTCGGGG 5'

5' AAAGTAAGCCCCGTC 3'
3' TTTCATTCGGGG 5'

5' AAAGTAAGCCCCGGT 3'
3' TTTCATTCGGGG 5'

5' AAAGTAAGCCCCGGC 3'
3' TTTCATTCGGGG 5'

5' AAAGTAAGCCCCGCT 3'
3' TTTCATTCGGGG 5'

5' AAAGTAAGCCCCGCC 3'
3' TTTCATTCGGGG 5'

5' AAAGTAAGCCCCCAT 3'
3' TTTCATTCGGGG 5'

5' AAAGTAAGCCCCCAC 3'
3' TTTCATTCGGGG 5'

5' AAAGTAAGCCCCCTT 3'
3' TTTCATTCGGGG 5'

5' AAAGTAAGCCCCCTC 3'
3' TTTCATTCGGGG 5'

5' AAAGTAAGCCCCCGT 3'
3' TTTCATTCGGGG 5'

5' AAAGTAAGCCCCCGC 3'
3' TTTCATTCGGGG 5'

5' AAAGTAAGCCCCCCT 3'
3' TTTCATTCGGGG 5'

5' AAAGTAAGCCCCCCC 3'
3' TTTCATTCGGGG 5'